# EUROPÄISCHE PATENTSCHRIFT

(11) **EP 0 892 070 B1**
(45) Veröffentlichungstag und Bekanntmachung des Hinweises auf die Patenterteilung: **11.06.2008**
(21) Anmeldenummer: 98113091.7
(22) Anmeldetag: 14.07.1998
(51) Int. Cl.: C12Q 1/68

(54) **Integriertes Verfahren und System zur Amplifizierung und zum Nachweis von Nukleinsäuren**
Integrated process and system for the amplification and detection of nucleic acids
Procédé et système intégrés pour l'amplification et la détection d'acides nucléiques

(30) Priorität: 15.07.1997 DE 19730359
(43) Veröffentlichungstag der Anmeldung: 20.01.1999
(73) Patentinhaber: Roche Diagnostics GmbH, 68305 Mannheim-Waldhof (DE)
(72) Erfinder: Kleiber, Jörg, Dr., 82377 Penzberg (DE); Tabiti, Karim, Dr., 81477 München (DE); Sagner, Gregor, Dr., 82377 Penzberg (DE)
(74) Vertreter: Weiss, Wolfgang

(56) Entgegenhaltungen:
- WO-A-97/07235
- US-A- 5 192 510
- US-A- 5 585 242
- TAVERRIARAKIS L ET AL: "Amplification and non-isotopic detection of specific DNA sequences in a single microtitre well" SERODIAGNOSIS AND IMMUNOTHERAPY IN INFECTIOUS DISEASE 1995 NETHERLANDS, Bd. 7, Nr. 4, 1995, Seiten 202-206, XP009010838 ISSN: 0888-0786
- SHINTARO KAWAI ET AL: "A SIMPLE METHOD OF DETECTING AMPLIFIED DNA WITH IMMOBILIZED PROBES ON MICROTITER WELLS" ANALYTICAL BIOCHEMISTRY, ACADEMIC PRESS, SAN DIEGO, CA, US, Bd. 209, Nr. 1, 15. Februar 1993 (1993-02-15), Seiten 63-69, XP000345602 ISSN: 0003-2697
- KELLER G H ET AL: "DETECTION OF HUMAN IMMUNODEFICIENCY VIRUS TYPE 1 DNA BY POLYMERASE CHAIN REACTION AMPLIFICATION AND CAPTURE HYBRIDIZATION IN MICROTITER WELLS" JOURNAL OF CLINICAL MICROBIOLOGY, WASHINGTON, DC, US, Bd. 29, 1. März 1991 (1991-03-01), Seiten 638-641, XP000319800 ISSN: 0095-1137
- WINN-DEEN E S: "Multi-mutation screening using PCR and ligation - principles and applications" TRENDS IN BIOTECHNOLOGY, ELSEVIER PUBLICATIONS, CAMBRIDGE, GB, Bd. 14, Nr. 4, 1. April 1996 (1996-04-01), Seiten 112-114, XP004035794 ISSN: 0167-7799

## Beschreibung

Die vorliegende Erfindung betrifft ein Verfahren und eine Vorrichtung, mit der eine integrierte Amplifikation und Detektion von Nukleinsäuren ermöglicht wird.

Heterogene Verfahren zum Nachweis von Nukleinsäuren enthalten den Schritt der Immobilisierung der nachzuweisenden Nukleinsäure. Dies kann beispielsweise direkt unter kovalenter Bindung der Nukleinsäure an eine Festphase oder über Kopplung der Nukleinsäure an einen Partner eines Affinitätspaares und Bindung des anderen Partners des Affinitätspaares an eine Festphase geschehen. Eine weitere Kopplungsmöglichkeit besteht auch durch Immobilisierung der nachzuweisenden Nukleinsäure mittels Hybridisierung mit einer festphasengebundenen, mindestens teilweise zu der nachzuweisenden Nukleinsäure komplementären sog. Fangsonde. Diese Fangsonde kann auf beliebige Weise an eine Festphase gebunden sein oder werden.

Beispiele für Verfahren, bei denen nachzuweisende Nukleinsäuren über Fangsonden immobilisiert werden, sind für die direkte Bindung in EP-A-0 079 139 und für die Bindung über ein Affinitätspaar in EP-A-0 192 168 beschrieben. Bei den dort beschriebenen Verfahrensführungen wird die nachzuweisende Nukleinsäure mit einer Nachweissonde, die ebenfalls mit der nachzuweisenden Nukleinsäure hybridisieren kann, hybridisiert und das gebildete Hybrid nach Entfernung überschüssiger Nachweissonde an der Festphase über eine Markierungsgruppe der Nachweissonde nachgewiesen.

Zum Nachweis selten vorkommender Nukleinsäuren in einer Probe wird üblicherweise eine targetspezifische Amplifikation durchgeführt. Ein solches Verfahren, bei dem beispielsweise eine Digoxigenin-Markierungsgruppe in die amplifizierte Nukleinsäure eingebaut wird, ist in WO92/06216 beschrieben.

Bei bisher gebräuchlichen Verfahren in der Nukleinsäurediagnostik erfolgten die Amplifikation und Detektion von Nukleinsäuren in getrennten Systemen, so daß die durch Amplifikation erzeugten Produkte vor dem Nachweis mit der Umgebung in Kontakt kamen. Auf diese Weise bestand ein hohes Risiko von Kreuzkontaminationen von falsch-positiven Ergebnissen. Außerdem mußten mehrere Pipettierschritte und mindestens ein Transferschritt erfolgen.

Aus US-A-5,210,015 ist ein kombiniertes Amplifikations- und Detektionsverfahren in einem geschlossenen System bekannt, wobei der Amplifikationsschritt in Gegenwart einer Nachweissonde erfolgt. Diese Nachweissonde bindet unter den Amplifikationsbedingungen an die Ziel-DNA und enthält eine Reporter- und eine Quencher-Gruppe. Durch die Nukleaseaktivität der Polymerase kann die Reportergruppe von der Sonde abgespalten werden, wodurch ein nachweisbares Signal entsteht. Dieses Verfahren hat jedoch den Nachteil einer geringen Sensitivität. Außerdem muß die Sonde im Hinblick auf ihre Sequenz und den Schmelzpunkt sehr sorgfältig ausgewählt werden.

Die Veröffentlichung Findlay et al. (Clin. Chem. 39 (1993), 1927-1933) offenbart ein automatisiertes Verfahren zur Amplifikation und Detektion von Nukleinsäuren, bei dem ein einziges Reaktionsgefäß mit mehreren separaten Abteilen verwendet wird, wobei das während der Amplifikation in einem ersten Abteil erzeugte Produktgemisch zum Nachweis in ein zweites separates Nachweisabteil überführt werden muß. Ein Nachteil dieses Verfahrens besteht darin, daß komplex aufgebaute Reaktionsgefäße oder/und aufwendige Überführungstechniken verwendet werden müssen.

WO93/10267 beschreibt ein Verfahren zur Amplifikation und Detektion von Nukleinsäuren, wobei während der Reaktion eine Nachweissonde vorhanden ist, die mindestens an ihrem 3'-Ende eine Elektrochemilumineszenz-Markierungsgruppe trägt, so daß sie nicht als Primer für die Amplifikation dienen kann. Ein Nachteil dieses Verfahrens besteht darin, daß eine aufwendige Konstruktion von Nachweissonden erforderlich ist. Darüber hinaus kann eine Störung der Amplifikationsreaktion durch die zugesetzte Nachweissonde nicht ausgeschlossen werden.

EP-A-0 628 568 offenbart ein Verfahren zur Amplifikation und nachfolgenden Immobilisierung bzw. Detektion von Nukleinsäuren durch Hybridisierung der Nukleinsäure mit einer Fangsonde, wobei die Fangsonde gegen enzymatische Verlängerung oder/und enzymatischen Abbau des gebildeten Hybrids geschützt ist. Die Fangsonde kann gegebenenfalls bereits während der Amplifikationsreaktion anwesend sein. Auch dieses Verfahren weist Nachteile dahingehend auf, daßdie Herstellung der geschützten Fangsonden aufwendig ist und daß eine Störung der Amplifikationsreaktion durch die Fangsonde trotzdem nicht ausgeschlossen werden kann.

WO 97/07235 beschreibt ein Verfahren zum Nachweis einer Nucleinsäure sequenz. Amplifikation und Nachweis der Amplifikationsprodukte erfolgen mit Primern und Sondern, wobei die Schmelztemperatur der Primer höher ist als die der Sondern.

US 5,585,242 beschreibt ein Verfahren zum Nachweis einer amplifizierten Nukleinsaüre durch totale interne Reflexion (TIR).

Eine der vorliegenden Erfindung zugrundeliegende Aufgabe war es daher, ein Verfahren zur Amplifikation und Detektion von Nukleinsäuren bereitzustellen, bei dem die Nachteile des Standes der Technik mindestens teilweise beseitigt sind. Insbesondere sollen Amplifikation und Detektion der Nukleinsäure in einer einzigen Vorrichtung erfolgen, die während des Verfahrens nicht geöffnet werden muß. Außerdem soll das Verfahren schnell, einfach handhabbar, automatisierbar und kostengünstig sein.

Die erfindungsgemäße Aufgabe wird gelöst durch ein Verfahren zum Nachweis einer Nukleinsäure, umfassend:
(a) Bereitstellen einer Probe, in der eine Nukleinsäure nachgewiesen werden soll,
(b) Amplifizieren der Nukleinsäure in einer Vorrichtung in Anwesenheit mindestens eines für die nachzuweisende Nukleinsäure spezifischen Nukleinsäure-Primers und unterschiedlichen, für die nachzuweisende Nukleinsäure spezifischen Fangsonden, wobei die Fangsonden an jeweils unterschiedlichen Positionen einer Innenoberfläche der Vorrichtung immobilisiert sind und derart ausgewählt werden, daß ein Hybrid aus Nukleinsäure und Fangsonde eine geringere Schmelztemperatur als ein Hybrid aus Nukleinsäure und Primer hat und wobei die Amplifikation unter solchen Bedingungen durchgeführt wird, daß ein Hybrid aus Nukleinsäure und Primer stabil ist, während ein Hybrid aus Nukleinsäure und Fangsonde instabil ist, und
(c) Nachweisen der Nukleinsäure durch Bindung der Amplifikationsprodukte an die immobilisierten Fangsonden mittels Evaneszenz-induzierter Fluoreszenz, und wobei der Nachweis unter Anwendung differentieller Hybridisierungsbedingungen zwischen nach zuweisender Nukleinsaüre und fangsonde erfolgt
wobei Amplifikation und Nachweis in einer einzigen Vorrichtung erfolgen, die während der Reaktion nicht geöffnet wird und wobei Fluoreszenzmarkierungen in das Amplifikationsprodukts eingebaut werden.

Durch das erfindungsgemäße Verfahren, wird durch Auswahl einer Fangsonde, die unter den Amplifikationsbedingungen nicht mit dem Primer oder/und der nachzuweisenden Nukleinsäure hybridisiert, eine gegenüber bekannten Verfahren deutlich verbesserte Sensitivität erreicht. Darüber hinaus besteht aufgrund der Verwendung einer geschlossenen integrierten Vorrichtung für Amplifikation und Detektion ein minimales Kontaminationsrisiko. Weiterhin ist das Verfahren einfach durchführbar und erfordert keinerlei Waschschritte. Das erfindungsgemäße Verfahren erlaubt darüber hinaus sowohl eine Multiplex-Amplifikation, z.B. durch Verwendung verschiedener, unterschiedlich markierter Primer als auch eine Multiplex-Detektion, z. B. durch Verwendung verschiedener Fangsonden an definierten Positionen der Oberfläche.

Schritt (a) des erfindungsgemäßen Verfahrens umfaßt das Bereitstellen von einer Probe, die die nachzuweisende Nukleinsäure enthält. Die Probe kann eine natürliche Probe, z.B. eine Körperflüssigkeit wie Blut, Serum, Plasma, Urin oder Cerebrospinalflüssigkeit, eine Gewebeprobe oder eine Kulturprobe, aber auch eine durch Prozesschritte aus einer natürlichen Probe hergestellte Probe sein. In einer bevorzugten Ausführungsform ist die Probe eine natürliche Probe biologischen Ursprungs, die zum Nachweis einer natürlich vorkommenden Nukleinsäure in der Nukleinsäurediagnostik dient.

Schritt (b) des erfindungsgemäßen Verfahrens umfaßt das Amplifizieren der Nukleinsäure in einer dafür geeigneten Vorrichtung. Die Amplifikation der nachzuweisenden Nukleinsäure kann auf jede bekannte Art und Weise erfolgen. Vorzugsweise erfolgt die Amplifikation unter Verwendung von einem oder mehreren Nukleinsäure-Primern, die mit der nachzuweisenden Nukleinsäure ausreichend komplementär sind, so daß sie bei den Amplifikationsbedingungen ein stabiles Hybrid mit der nachzuweisenden Nukleinsäure bilden können. Diese Primer werden vorzugsweise durch ein ebenfalls im Reaktionsgemisch anwesendes Enzym, z.B. eine DNA-Polymerase, verlängert. Beispiele für geeignete Amplifikationsverfahren sind die Ligase-Kettenreaktion (LCR) und insbesondere die Polymerase-Kettenraktion (PCR). Weiterhin ist bevorzugt, daß die Amplifikation mit einem thermostabilen Enzym als thermocyclisch geführte Reaktion in mehreren, z.B. 20 bis 30 Zyklen durchgeführt wird.

Der beim erfindungsgemäßen Verfahren anwesende Nukleinsäure-Primer ist ein Oligonukleotid oder ein modifiziertes Oligonukleotid, welches an seinem 3'-Ende einer enzymatischen Extension zugänglich ist. Die Länge des Nukleinsäureprimers kann je nach Erfordernissen über einen weiten Bereich variiert werden und beträgt vorzugsweise 15 bis 50 Nukleotide. Die während der Amplifikation ebenfalls anwesenden Fangsonden können ebenfalls ein Oligonukleotid oder ein modifiziertes Oligonukleotid, z.B. eine peptidische Nukleinsäure sein. Vorzugsweise werden Fangsonden verwendet, die kein für eine enzymatische Extension zugängliches 3'-Ende aufweisen. Die Fangsonden sind an einer Innenoberfläche der integrierten Amplifikations- und Detektionsvorrichtung immobilisiert. Die Immobilisierung kann auf jede bekannte Art und Weise, z.B. durch kovalente Kopplung oder durch hochaffine biologische Wechselwirkungen erfolgen.

Ein Beispiel für ein geeignetes Immobilisierungsverfahren von Nukleinsäuren besteht darin, daß unter Verwendung der Langmuir-Blodgett Technik Cinnamoylbutylether-Cellulose-Monoschichten auf eine Innenoberfläche der Detektionsvorrichtung transferiert werden. Nach dem Filmtransfer werden die Monoschichten durch Photopolymerisation der olefinischen Doppelbindungen stabilisiert. Im Film verbleibende Doppelbindungen können durch Behandlung mit OₛO₄ und NalO₄ in Aldehydgruppen zur nachfolgenden kovalenten Kopplung von Streptavidin oder anderen Substanzen, die zur Immobilisierung von Bindepartnern durch hochaffine biologische Wechselwirkungen in der Lage sind, z.B. Antikörpern oder Lectinen, überführt werden. Anschließend wird der Film mit der kovalent zu koppelnden Substanz unter geeigneten Bedingungen, z.B. mit einer wässrigen Streptavidin-Lösung (10 µg/ml) in Kontakt gebracht. Gegebenenfalls kann anschließend ein Blockierungsschritt mit einer inerten Substanz, z.B. Rinderserumalbumin, erfolgen.

Auf dieser Oberfläche können Fangsonden immobilisiert werden, die den komplementären Bindepartner für die hochaffine biologische Wechselwirkung, z.B. Biotin, enthalten. Diese Immobilisierung kann durch Inkontaktbringen der Oberfläche mit einer die Fangsonden enthaltenden Lösung (Konzentration z.B. 200 nmol/l) bei 37°C für 1 h erfolgen. Die genaue Durchführung der Schritte zur Herstellung einer mit einer Fangsonde beschichteten Oberfläche ist beispielsweise bei Furch et al. (SPIE 2928 (1996), 220-226) beschrieben.

Für das erfindungsgemäße Verfahren ist es wesentlich, daß die Primerextension bei einer Temperatur erfolgt, die größer als die Schmelztemperatur eines Hybrids zwischen der immobilisierten Fangsonde und der durch das Verfahren nachzuweisenden Nukleinsäure ist. Auf diese Weise wird eine signifikante Verbesserung der Testspezifität und Sensitivtät erreicht.

Besonders bevorzugt erfolgt die Amplifikation bei einer Temperatur, die um mindestens 2°C, insbesondere mindestens um 5°C und am meisten bevorzugt mindestens um 8°C höher als der Schmelzpunkt eines Hybrids zwischen Fangsonde und nachzuweisender Nukleinsäure liegt. Die Auswahl geeigneter Fangsondensequenzen kann ohne weiteres unter Bezugnahme auf bekannte Tabellen für die Stabilität von Nukleinsäurehybriden, z.B. Nukleinsäure-Nukleinsäure-Hybriden oder Hybriden zwischen Nukleinsäuren und peptidischen Nukleinsäuren erfolgen.

Ein weiterer Vorteil des erfindungsgemäßen Verfahrens besteht darin, daß eine Multiplex-Amplifikation durchgeführt werden kann. Bei einer solchen Multiplex-Amplifikation können beispielsweise mehrere unterschiedliche Primer verwendet werden, die jeweils mit unterschiedlichen, nebeneinander nachweisbaren Markierungsgruppen versehen sind. Auf diese Weise können beispielsweise miteinander kompetierende Primer für eine allelspezifische Amplifikation der nachzuweisenden Nukleinsäure eingesetzt werden, wie etwa von Lee und Hall (Annals of the Entomological Society of America 89 (1996), 20-27) beschrieben ist.

Schritt (c) des erfindungsgemäßen Verfahrens umfaßt die Detektion des in Schritt (b) erzeugten Amplifikationsprodukts durch Bindung an die immobilisierten Fangsonden. Der Nachweisschritt wird zweckmäßigerweise bei einer geringeren Temperatur als der Amplifikationsschritt durchgeführt, d.h. bei einer Temperatur, bei der ein stabiles Hybrid zwischen nachzuweisender Nukleinsäure und Fangsonde gebildet wird.

Erfindungsgemäß wird das Nachweis der Bindung einer Nukleinsäure an die immobilisierten Fangsonden durch Evaneszenz-induzierte Fluoreszenz. Hierzu durchgeführt werden die Fangsonden bevorzugt auf einer optisch transparenten Innenoberfläche der Vorrichtung, z.B. der Vertiefung einer Mikrotiterplatte immobilsiert. Während der Amplifikation werden dann fluoreszenzmarkierte Nukleotide in das Amplifikationsprodukt eingebaut. Dies kann entweder durch Verwendung fluoreszenzmarkierter Primer oder/und fluoreszenzmarkierter monomerer Dideoxynukleotidtriphosphate erfolgen. Der Nachweis einer Evaneszenz-induzierten Fluoreszenz kann durch ein Mikroskop, z.B. ortsaufgelöst durch konfokale Mikroskopie, durch einen Scanner oder eine CCD-Kamera erfolgen. Es sind zeitaufgelöste Messungen oder Realzeitmessungen möglich.

Die Detektion erfolgt beim erfindungsgemäßen Verfahren als Multiplex-Detektion. Hierbei werden mehrere unterschiedliche Fangsonden verwendet, die jeweils an unterschiedlichen Positionen auf der Oberfläche der integrierten Amplifikations- und Detektionsvorrichtung immobilisiert sind. Die Detektion erfolgt unter Anwendung differenzieller Hybridisierungsbedingungen zwischen nachzuweisender Nukleinsäure und Fangsonden, z.B. durch kontrollierte Temperaturerhöhung Auf diese Weise kann beispielsweise eine Mutationsanalyse der nachzuweisenden Nukleinsäure durchgeführt werden.

Beispiele für optisch transparente Materialien, an welche die Fangsonden immobilisiert werden können, sind organische oder anorganische optisch transparente Materialien wie etwa Glas, Quarz, Silikon, Kunststoffe wie Polycarbonat, Acrylpolymere oder Polystyrol. Die Immobilisierung der Fangsonden an die Oberfläche der Reaktionsvorrichtung ist eine thermostabile "Immobilisierung" so daß während der bei der Amplifikationsreaktion herrschenden Temperaturen keine signifikante Ablösung der Fangsonde eintritt. Einerseits können die Sonden durch kovalente Immobilisierungstechniken an der Festphase fixiert werden. Andererseits und bevorzugt kann die Immobilisierung über ein hochaffines Bindepaar, z.B. Streptavidin/Biotin erfolgen.

Der Nachweis von Nukleinsäuren durch Evaneszenz-induzierte Fluoreszenz ist bereits bekannt (vgl. z.B. EP-A-0 245 206; WO96/09532). Überraschenderweise wurde jedoch festgestellt, daß durch das erfindungsgemäße Verfahren, welches eine Kombination einer Amplifikationsreaktion in Gegenwart von immobilisierten, nicht interferierenden Fangsonden und den Nachweis des Amplifikationsprodukts durch Evaneszenz-induzierte Fluoreszenz umfaßt, besonders sensitive und zuverlässige Testergebnisse erhalten werden können.

Das erfindungsgemäße Verfahren kann durchgeführt werden unter Verwendung eines Reagenzienkits zum Nachweis von Nukleinsäuren umfassend:
(a) eine Vorrichtung zur Amplifikation und Detektion von Nukleinsäuren mit mindestens einer auf mindestens einer Innenoberfläche davon immobilisierten Fangsonde,
(b) mindestens einen Nukleinsäure-Primer,
(c) gegebenenfalls Deoxyribonukleotide und
(d) gegebenenfalls ein zur Extension des Nukleinsäure-Primers geeignetes Enzym,
wobei die Fangsonde derart ausgewählt ist, daß ein Hybrid aus nachzuweisender Nukleinsäure und Fangsonde eine geringere Schmelztemperatur als ein Hybrid aus nachzuweisender Nukleinsäure und Primer hat.

Die Vorrichtung stellt ein abgeschlossenes System dar, welches eine integrierte Amplifikation und Detektion einer in einer Probe enthaltenen Nukleinsäure ermöglicht. Beispiele für geeignete Amplifikations- und Detektionsvorrichtungen sind Reaktionsgefäße, z.B. Mikroreaktionsgefäße mit einem Volumen bis 100 µl, wie etwa Mikrotiterplatten. Darüber hinaus kommen selbstverständlich auch andere Vorrichtungen, wie etwa Biosensoren, z.B. Chips, in Frage. Die Fangsonden sind vorzugsweise auf einer optisch transparenten Oberfläche der Vorrichtung immobilisiert. Der Kit enthält vorzugsweise eine Fluoreszenzmarkierung, beispielsweise einen markierten Nukleinsäure-Primer oder/und markierte Deoxyribonukleotide. Das im Kit enthaltene Enzym ist vorzugsweise eine thermostabile DNA-Polymerase, z.B. die Taq-Polymerase.

Ein weiterer verwenbarer Reagenzienkit umfasst.
(a) eine Vorrichtung zur Amplifikation und Detektion von Nukleinsäuren mit mindestens einer auf einer transparenten Innenoberfläche davon immobilisierten Fangsonde,
(b) mindestens einen Nukleinsäuren-Primer,
(c) gegebenenfalls Deoxyribonukleotide,
(d) gegebenenfalls ein zur Extension des Nukleinsäure-Primers geeignetes Enzym,
(e) eine Fluoreszenzmarkierung und
(f) gegebenenfalls Mittel zum Nachweis von Amplifikationsprodukten durch Evaneszenz- induzierte Fluoreszenz.

## Patentansprüche

1. Verfahren zum Nachweis einer Nukleinsäure umfassend:
(a) Bereitstellen einer Probe, in der eine Nukleinsäure nachgewiesen werden soll,
(b) Amplifizieren der Nukleinsäure in einer Vorrichtung in Anwesenheit mindestens eines für die nachzuweisende Nukleinsäure spezifischen Nukleinsäure-Primers und unterschiedlichen, für die nachzuweisende Nukleinsäure spezifischen Fangsonden, wobei die Fangsonden an jeweils unterschiedlichen Positionen einer Innenoberfläche der Vorrichtung immobilisiert sind und derart ausgewählt werden, dass ein Hybrid aus Nukleinsäure und Fangsonde eine geringere Schmelztemperatur als ein Hybrid aus Nukleinsäure und Primer hat und wobei die Amplifikation unter solchen Bedingungen durchgeführt wird, dass ein Hybrid aus Nukleinsäure und Primer stabil ist, während ein Hybrid aus Nukleinsäure und Fangsonde instabil ist, und
(c) Nachweisen der Nukleinsäure durch Bindung der Amplifikationsprodukte an die immobilisierten Fangsonden mittels Evaneszenzinduzierter Fluoreszenz, und wobei der Nachweis unter Anwendung differentieller Hybridisierungsbedingungen zwischen nachzuweisender Nukleinsäure und Fangsonde erfolgt,
wobei Amplifikation und Nachweis in einer einzigen Vorrichtung erfolgen, die während der Reaktion nicht geöffnet wird und
wobei Fluoreszenzmarkierungen in das Amplifikationsprodukt eingebaut werden.

2. Verfahren nach Anspruch 1,
**dadurch gekennzeichnet,**
**dass** man die Reaktion in einer Mikrotiterplattenvertiefung durchführt.

3. Verfahren nach einem der Ansprüche 1 bis 2,
**dadurch gekennzeichnet,**
**dass** die Fangsonden auf einer optisch transparenten Innenoberfläche der Vorrichtung immobilisiert werden.

4. Verfahren nach einem der Ansprüche 1 bis 3,
**dadurch gekennzeichnet,**
**dass** man eine immobilisierte Fangsonde ausgewählt aus Nukleinsäuren und peptidischen Nukleinsäuren verwendet.

5. Verfahren nach einem der Ansprüche 1 bis 4,
**dadurch gekennzeichnet,**
**dass** die Anwendung differentieller Hybridisierungsbedingungen durch kontrollierte Temperaturerhöhung erfolgt.

## Claims

1. Method for detection of a nucleic acid, comprising:
(a) providing a sample in which it is intended to detect a nucleic acid,
(b) amplifying the nucleic acid in a device in the presence of at least one nucleic acid primer which is specific for the nucleic acid to be detected and different capture probes which are specific for the nucleic acid to be detected, the capture probes being immobilized at respectively different positions of an inner surface of the device and selected in such a way that a hybrid of nucleic acid and capture probe has a lower melting temperature than a hybrid of nucleic acid and primer and wherein the amplification is carried out under such conditions that a hybrid of nucleic acid and primer is stable whereas a hybrid of nucleic acid and capture probe is unstable, and
(c) detecting the nucleic acid by the binding of the amplification products to the immobilized capture probes by means of evanescence-induced fluorescence and wherein the detection is effected using differential hybridization conditions between the nucleic acid to be detected and the capture probe,
wherein amplification and detection take place in a single device which is not opened during the reaction and wherein fluorescent labels are incorporated into the amplification product.

2. Method according to Claim 1, **characterized in that** the reaction is carried out in a microtitre plate well.

3. Method according to either of Claims 1 to 2, **characterized in that** the capture probes are immobilized on an optically transparent inner surface of the device.

4. Method according to any one of Claims 1 to 3, **characterized in that** an immobilized capture probe selected from nucleic acids and peptidic nucleic acids is used.

5. Method according to any one of Claims 1 to 4, **characterized in that** the using of differential hybridization conditions is effected by controlled temperature increase.

## Revendications

1. Procédé pour mettre en évidence un acide nucléique comprenant :
(a) la fourniture d'un échantillon dans lequel un acide nucléique doit être mis en évidence,
(b) l'amplification de l'acide nucléique dans un dispositif en présence d'au moins une amorce d'acide nucléique spécifique de l'acide nucléique qui doit être mis en évidence et de différentes sondes de capture spécifiques de l'acide nucléique qui doit être mis en évidence, où les sondes de capture sont immobilisées à différentes positions d'une surface interne du dispositif et sont choisies de telle manière qu'un hybride d'acide nucléique et de sonde de capture a une plus faible température de fusion qu'un hybride d'acide nucléique et d'amorce et où l'amplification est conduite dans des conditions telles qu'un hybride d'acide nucléique et d'amorce est stable, tandis qu'un hybride d'acide nucléique et de sonde de capture est instable, et
(c) la mise en évidence de l'acide nucléique par liaison des produits d'amplification aux sondes de capture immobilisées à l'aide de fluorescence induite par évanescence, et où la mise en évidence a lieu en utilisant des conditions d'hybridation différentielle entre l'acide nucléique qui doit être mis en évidence et la sonde de capture,
où l'amplification et la mise en évidence ont lieu dans un seul dispositif qui n'est pas ouvert pendant la réaction et
où des marquages de fluorescence sont incorporés dans le produit d'amplification.

2. Procédé selon la revendication 1 **caractérisé en ce que** l'on conduit la réaction dans un puits d'une microplaque de titrage.

3. Procédé selon l'une des revendications 1 à 2 **caractérisé en ce que** les sondes de capture sont immobilisées sur une surface interne optiquement transparente du dispositif.

4. Procédé selon l'une des revendications 1 à 3 **caractérisé en ce que** l'on utilise une sonde de capture immobilisée choisie parmi les acides nucléiques et les acides nucléiques peptidiques.

5. Procédé selon l'une des revendications 1 à 4 **caractérisé en ce que** l'utilisation de conditions d'hybridation différentielle a lieu par augmentation contrôlée de la température.
